(19) **Europäisches Patentamt**
**European Patent Office**
**Office européen des brevets**

(11) **EP 1 031 358 B1**

(12) **FASCICULE DE BREVET EUROPEEN**

(45) Date de publication et mention
de la délivrance du brevet:
**11.07.2007 Bulletin 2007/28**

(51) Int Cl.:
*A61M 5/168* (2006.01)    *F04B 43/08* (2006.01)

(21) Numéro de dépôt: **00103516.1**

(22) Date de dépôt: **18.02.2000**

(54) **Procédé de contrôle d'une pompe linéaire péristaltique**

Kontrollverfahren für peristaltische Pumpe

Control procedure for a linear peristaltic pump

(84) Etats contractants désignés:
**AT BE CH CY DE DK ES FI FR GB GR IE IT LI LU MC NL PT SE**

(30) Priorité: **23.02.1999 FR 9902390**

(43) Date de publication de la demande:
**30.08.2000 Bulletin 2000/35**

(73) Titulaire: **Fresenius Vial SAS**
**38590 Brézins (FR)**

(72) Inventeurs:
 • **Jhuboo, Abdel Nasser**
 **38590 Saint Etienne de Saint Geoirs (FR)**

 • **Rondelet, Jean-Claude**
 **38960 Saint Etienne de Crossey (FR)**

(74) Mandataire: **Vièl, Christof**
**Cabinet Vièl**
**1, rue des Bleuets,**
**BP 18**
**57520 Grosbliederstroff (FR)**

(56) Documents cités:
**EP-A- 0 315 312      EP-A- 0 431 310**
**US-A- 4 840 542      US-A- 5 103 211**
**US-A- 5 336 053**

**Description**

L'invention concerne un procédé de contrôle d'un dispositif de pompage comportant une pompe à doigts munie d'un tube souple.

Il est courant en médecine d'utiliser des dispositifs de perfusion dit goutte-à-goutte. Il s'agit d'administrer à un patient une solution à un débit constant relativement lent. Ces goutte-à-goutte sont constitués d'une part d'un tube reliant le récipient contenant la solution au patient et d'autre part d'une pompe volumétrique contrôlant le débit. Il est indispensable pour la sécurité du patient de contrôler l'écoulement de la solution tout au long du dispositif de goutte-à-goutte. Pour cela, il est courant d'associer à la pompe un détecteur de gouttes constitué généralement par un détecteur optique connecté à la chambre à gouttes du dispositif de goutte-à-goutte.

Ces détecteurs de gouttes assurent un haut niveau de sécurité. Ils permettent de détecter :

- les situations de sous-débit (under-flow) où aucune goutte ne se forme, en raison par exemple d'une occlusion du tube, d'un mauvais positionnement du tube dans la pompe ou d'un inversement du sens du tube dans la pompe, ainsi que
- les situations de surdébit (over-flow) où les gouttes se suivent à un rythme trop rapide (le rythme normal étant approximativement de 20 gouttes min) ou tellement rapide qu'il se forme un filet de solution, par exemple en raison d'une rupture du tube, d'un mauvais positionnement du tube dans la pompe ou d'un défaut de la pompe.

Ces détecteurs de gouttes ont cependant l'inconvénient d'être très sensibles et de déclencher de fausses alarmes. Il suffit parfois que la chambre à gouttes soit inclinée ou que des gouttelettes soient présentes sur la paroi de la chambre à gouttes pour générer de fausses informations.

On connaît du document EP 0 315 312 un procédé pour détecter une limitation partielle dans un tube souple reliant une source de liquide à une pompe à doigts pour intraveineuse. Le procédé prévoit d'obturer le tube avec un des doigts de la pompe, de mesurer dans la pompe la pression régnant dans le tube en amont de l'occlusion, de déterminer si la valeur dépasse une valeur limite déterminée et de déclencheur une alarme dans ce cas. Ce procédé permet de détecter une éventuelle limitation dans le tube en amont du point d'occlusion. Il ne permet cependant pas de détecter les éventuelles limitations en aval du point d'obturation, ni de détecter une éventuelle défaillance de la pompe, notamment une éventuelle fracture de la plaque d'appui de la pompe. Il n'est pas possible de contrôler l'ensemble du cycle de pompage.

On connaît du document US-A-4 840 542 un procédé de mesure de la pression dans une pompe à cassette reliant une source de liquide à un patient La pompe comprend un segment d'entrée, une chambre d'alimentation, un segment de transfert, une chambre de pompage et un segment de sortie. Elle est également munie d'une première, d'une seconde et d'une troisième valve permettant d'obturer le segment d'entrée, le segment de transfert et le segment de sortie respectivement. Un élément de pompe à pétales agit sur la chambre de pompage et un élément de pompage agit sur la chambre d'alimentation. Ces deux éléments de pompage sont reliés par une barre pivotante de sorte que lorsqu'un des éléments est déplacé dans un sens, l'autre est déplacé dans l'autre. Par ailleurs, la première et la deuxième valve agissent en tandem : quand l'une est ouverte, l'autre est fermée. Une cellule détecte la pression régnant dans la chambre de pompage. Lors du pompage, le second élément de pompage est fermé, mais les deuxième et troisième valves restent tout d'abord fermées de sorte qu'il se forme une surpression dans la chambre de pompage. Cette surpression est mesurée. Si la surpression n'est pas conforme à la valeur attendue, il se déclenche une alarme. Dans le cas contraire, la troisième valve est ouverte de sorte que le liquide contenu dans la chambre de pompage est délivré au patient. La cellule continue à mesurer la pression dans la chambre de pompage. Une surpression indiquerait une obturation du tube en aval de la pompe. Lorsque le deuxième élément de pompage appuie sur la chambre de pompage, le premier élément de pompage se rétracte provoquant l'entrée de liquide dans la chambre d'alimentation. Lorsque la pompe est en bout de course, la première et la troisième valves sont fermées tandis que la deuxième valve est ouverte. Un mouvement inverse rapide des éléments de pompage provoque le transfert du liquide de la chambre d'alimentation dans la chambre de pompage, et le cycle peut recommencer. Ce procédé, en provoquant une obturation en aval de la pompe permet de détecter que la pompe est correctement alimentée. En mesurant l'évolution de la pression dans la pompe lors de l'évacuation du liquide hors de la chambre de pompage, il permet de détecter une occlusion en aval de la pompe. Par contre, ce procédé ne permet pas de contrôler l'ensemble du cycle. De plus, il ne peut pas être appliqué à une pompe traversée directement par un tube.

L'objectif de l'invention est donc de développer un procédé de contrôle du dispositif de pompage comportant une pompe à doigts munie d'un tube souple tels que ceux utilisés pour les goutte-à-goutte qui ne présente pas les inconvénients du procédé connu et mis en oeuvre sous forme de détecteur de gouttes.

Cet objectif est atteint par le procédé conforme à l'invention dans lequel le tube souple est obturé à l'aide d'un dispositif d'obturation situé en aval de la pompe, puis la pompe est mise en marche et la pression instantanée $P_I$ est mesurée dans la section du tube située entre la pompe et le dispositif d'obturation. Avant de commencer la perfusion, on obture le tube de perfusion en aval de la pompe et on laisse la pompe travailler contre cet obstacle. Il doit se développer dans la section du tube située entre la pompe et l'obturation une surpression dont la valeur instantanée est mesurée. Si la pompe est défectueuse, il arrive au cours du processus de pompage un moment où il n'y a pas de zone d'occlusion. Dans ce cas, la surpression diminue en raison d'un reflux de liquide le temps que la zone d'occlusion réapparaisse. Cette perte de charge peut être détectée en mesurant la pression instantanée $P_I$. Lorsque la pompe est utilisée pour une perfusion, un tel défaut entraîne pendant un certain temps se répétant cycliquement un débit non contrôlé de la solution. De même, il ne se forme aucune surpression en cas d'occlusion du tube en amont de la pompe, en cas de mauvais positionnement du tube dans la pompe ou en cas d'inversement du sens du tube dans la pompe.

Il est conforme à l'invention que les étapes suivantes soient réalisées :

a) la pompe, réglée sur un débit proche du débit maximum, effectue un nombre de cycles prédéterminé au moins égal à 1 puis elle est arrêtée ;
b) une alarme est déclenchée si la pression instantanée $P_I$, mesurée tout au long de l'étape a), n'atteint pas une valeur seuil $P_1$ au plus tard à la fin de l'étape précédente.

Il est indispensable, pour réaliser un contrôle suffisant, que le cycle complet de la pompe soit contrôlé. Pour des raisons de sécurité, il sera préférable de lui faire exécuter un peu plus d'un cycle, par exemple 1,1 cycle. Si la pompe est défectueuse, l'augmentation de la pression n'est pas constante et la pompe ne permet pas d'atteindre la valeur seuil à la fin de la rotation programmée. La valeur seuil est calculée statistiquement pour chaque type de pompe.

Un autre développement de l'invention prévoit de réaliser les étapes suivantes, si aucune alarme n'est déclenchée lors de l'étape b) :

c) la pompe, réglée sur un débit intermédiaire, effectue un nombre de cycles prédéterminé au moins égal à 1 puis elle est arrêtée ;
d) la pression minimum $P_{min}$ et la pression maximum $P_{max}$ atteintes lors de l'étape précédente sont mesurées ;
e) une alarme est déclenchée si la pression minimum $P_{min}$ ou la pression maximum $P_{max}$ déterminées à l'étape d) n'atteignent pas les valeurs seuil $P_2$ et $P_3$ respectivement.

Le débit intermédiaire choisi lors de l'étape c) est un débit qui permet de détecter toute perte de charge tout en assurant une durée de contrôle relativement courte (< 10 s).

Un autre développement avantageux du procédé prévoit au cours d'une étape f) de déclencher une alarme si la pression instantanée $P_1$ atteint une valeur seuil $P_4$ au cours de l'étape c). Il s'agit ici de détecter la formation d'une surpression excessive susceptible de détériorer le tube ou la pompe.

Afin de remettre le dispositif de perfusion en état de fonctionnement normal, il est prévu au cours d'une étape g) de faire effectuer à la pompe en sens inverse le nombre exact de cycles qu'elle a réalisé lors des étapes précédentes, puis de réouvrir le dispositif d'obturation à la fin du procédé si aucune alarme n'a été déclenchée. Cette précaution permet de réaliser le procédé de contrôle aussi bien en dehors qu'au cours d'une perfusion. Ceci est également nécessaire si le procédé doit être automatisé.

Ce procédé est particulièrement bien adapté aux pompes à doigts. Il permet entre autres de détecter une éventuelle fracture de la plaque d'appui de la pompe.

Un dispositif de mise en oeuvre du procédé de contrôle conforme à l'invention comporte un tube souple, une pompe dans laquelle le tube souple est introduit, un dispositif d'obturation situé en aval de la pompe et un détecteur de pression situé entre la pompe et le dispositif d'obturation.

Dans un mode de réalisation avantageux, le dispositif de mise en oeuvre du procédé comporte un détecteur de pression situé en amont de la pompe. Ce deuxième détecteur de pression permet de détecter une occlusion ou une rupture du tube en amont de la pompe aussi bien lors du procédé de contrôle lui-même qu'au cours de la perfusion.

Il est conforme à l'invention que le dispositif de mise en oeuvre du procédé comporte une unité de commande permettant le contrôle de l'ouverture et de la fermeture du dispositif d'obturation, le réglage du débit de la pompe et la mise en marche de la pompe pendant le nombre de cycles déterminé lors des étapes a) et c), l'analyse des valeurs mesurées de la pression instantanée $P_I$ pour les étapes b), d), e) et f), l'arrêt de la pompe lorsqu'une alarme est générée et permettant de faire effectuer en sens inverse à la pompe autant de cycles qu'il y en a eu lors des étapes précédentes. Ainsi équipé, le dispositif de mise en oeuvre du procédé peut travailler automatiquement. L'unité de commande provoque la fermeture du dispositif d'obturation du tube, règle le débit de la pompe à une valeur proche du maximum puis lui fait effectuer 1,1 cycle par exemple tout en analysant les valeurs fournies par le détecteur de pression. Lors de la deuxième partie du procédé, elle règle le débit de la pompe sur une valeur intermédiaire puis fait effectuer à la pompe 1,1 cycle par exemple tout en analysant les valeurs de la pression instantanée $P_I$ fournies par le détecteur de pression. Cette même unité de contrôle déclenche les alarmes en cas de valeurs hors norme. Si au contraire le contrôle a été effectué de façon satisfaisante, elle fait tourner la pompe en sens inverse d'autant de cycles qu'elle lui en a fait faire lors des étapes précédentes, puis réouvre le dispositif d'obturation. Il peut être possible de programmer cette unité de contrôle pour qu'elle effectue le procédé de contrôle au début de chaque perfusion et éventuellement au cours de celle-ci.

Afin d'augmenter la sécurité, il peut être avantageux de ne permettre l'introduction du tube dans la pompe que dans un sens prédéterminé. Pour cela on pourra par exemple munir le tube d'un dispositif ayant la forme d'une flèche indiquant le sens d'écoulement du liquide telle que la pince décrite dans la demande de brevet FR 99 01132.

Un exemple de procédé conforme à l'invention et un dispositif de mise oeuvre de ce procédé sont décrits ci-dessous.

La figure 1 représente un dispositif de mise en oeuvre du procédé de contrôle conforme à l'invention ;

La figure 2 représente un dispositif d'obturation ;

Le diagramme 1 représente le courant mesuré dans le moteur du dispositif d'obturation lors de l'ouverture ou la fermeture de celui-ci ;

Le diagramme 2 représente différentes courbes de pression instantanée mesurées lors du procédé de contrôle conforme à l'invention.

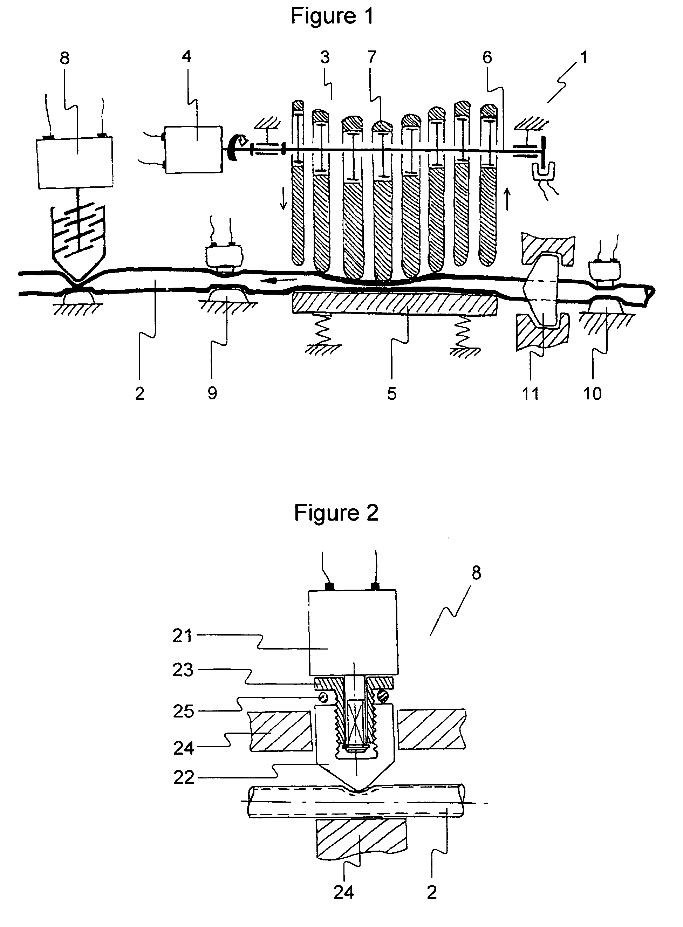

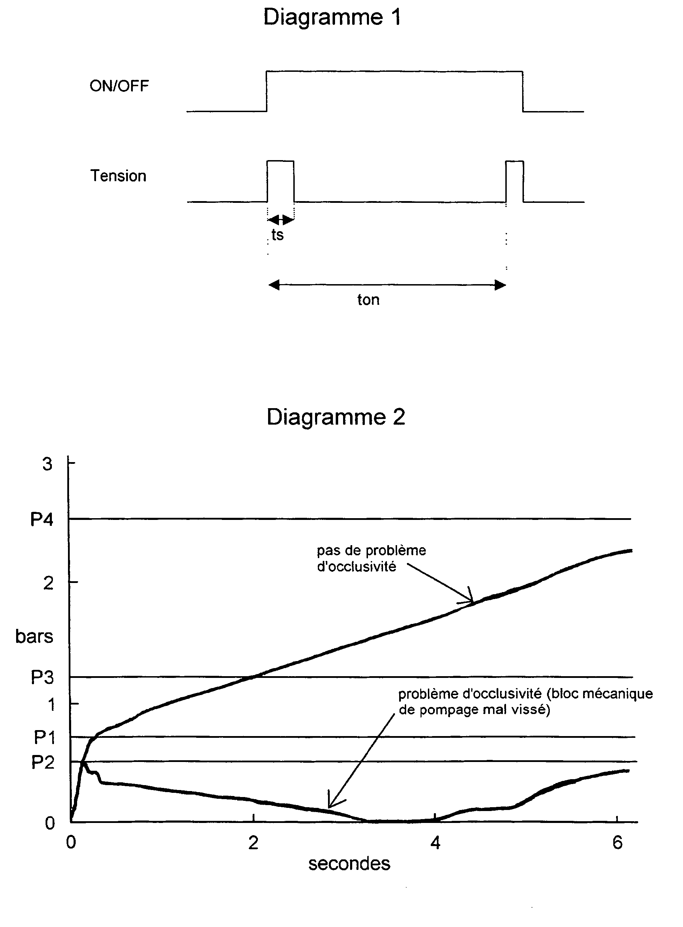

Comme le montre la figure 1, le dispositif de mise en oeuvre du procédé de contrôle (1) conforme à l'invention est constitué d'un tube souple (2) placé dans une pompe à doigts (3) actionnée par un moteur (4) et munie d'une plaque d'appui (5), d'un arbre à cames (6) et de doigts (7). Il comporte également un dispositif d'obturation (8) placé en aval de la pompe (3), un détecteur de pression (9) situé entre la pompe (3) et le dispositif d'obturation (8), un deuxième détecteur de pression (10) situé en amont de la pompe (3) et une pince automatique (11) en forme de flèche.

Les détecteurs de pression fournissent un signal de tension directement proportionnel à la pression régnant dans le tube. Ils sont calibrés à deux pressions distinctes, par exemple à la pression 0 bar ($P_{0bar}$) et à la pression 1 bar ($P_{1bar}$).

Le dispositif d'obturation (8) peut être composé d'un moteur à courant continu (21), d'une tête (22) reliée au moteur (21) par un dispositif à vis (23) comme le montre la figure 2. Pour obturer le tube (2), le moteur (21) tourne par exemple dans le sens des aiguilles d'une montre provoquant le déplacement par translation de la tête (22). Lorsque le tube est comprimé par la tête (22) contre le boîtier (24) du dispositif d'obturation (8), il est obturé. A ce moment là, le moment du moteur augmente et le courant dans le moteur augmente proportionnellement. Ce courant est mesuré et lorsqu'il atteint une valeur limite, le moteur est éteint. Pour ouvrir le tube, le moteur (21) tourne dans le sens inverse. Un anneau en caoutchouc (25) situé sur le dispositif à vis (23) en fin de course de la tête (22) se trouve comprimé lorsque la tête (22) est entièrement rentrée. Le courant dans le moteur augmente et lorsqu'il atteint une valeur limite le moteur est éteint.

Le moteur (21) est dirigé par un pont en H. Lorsque le moteur est mis en marche, le courant dans la bobine est important puis il diminue après environ 100 ms. Le microprocesseur analyse la valeur du courant et génère une alarme si le temps nécessaire à la diminution du courant (ts) est supérieur à 300 ms, signe d'un dysfonctionnement du dispositif d'obturation (8). En fin de course de la tête (22), soit lorsqu'elle comprime le tube souple (2) dans sa position sortie, soit lorsqu'elle comprime l'anneau de caoutchouc (25) dans sa position rentrée, le microprocesseur détecte à nouveau une augmentation de courant et il arrête le moteur avec un signal on/off. Si le temps (ton) nécessaire pour atteindre cette augmentation de courant dépasse 5 s, le microprocesseur déclenche une alarme. Ce procédé de contrôle est schématisé dans le diagramme 1.

Lors de l'exécution du procédé décrit dans l'exemple retenu ici, l'unité de contrôle, non représentée à la figure 1, provoque la fermeture du dispositif d'obturation (8). La pression initiale $P_0$ dans le tube est mesurée par le détecteur de pression (9). La pompe (3) est réglée sur un débit proche du débit maximum, par exemple 1 000 ml/h, puis le moteur est mis en marche pour 1,1 tour. Cette opération dure environ 0,4 s. Le moteur provoque la rotation de l'arbre à cames (6) qui entraîne les doigts (7) les uns après les autres dans un mouvement de translation vertical. En coinçant le tube entre les doigts (7) et la plaque d'appui (5), la pompe (3) provoque le déplacement de la solution. La pression instantanée $P_i$ augmente dans la partie située entre la pompe (3) et le dispositif d'obturation (8). Elle est mesurée par le détecteur de pression (9) qui produit un signal de tension. L'unité de contrôle du dispositif de mise en oeuvre (1) compare la valeur de la pression instantanée à une valeur seuil. L'équation suivante (A) doit être satisfaite au plus tard à la fin du 1,1 tour:

$$(A) \qquad (P_i - P_0) \ \geq P_1 \qquad\qquad \text{avec } P_1 = Q_1 \times (P_{1bar} - P_{0bar})$$

Le paramètre $Q_1$ est déterminé statistiquement à l'aide de plusieurs pompes du même type. Il est de l'ordre de 0,7. Dès qu'un cycle complet a été réalisé et que l'équation (A) est vérifiée, le moteur est arrêté. Si l'équation (A) n'est pas vérifiée après 1,1 tour de moteur, l'unité de contrôle déclenche une alarme et le procédé est arrêté. En effet, si la pression n'atteint pas la valeur seuil, ceci indique que la pompe ne fonctionne pas correctement. C'est le cas par exemple lorsque le plaque d'appui (5) est brisée ou lorsqu'un doigt (7) de la pompe (3) ne provoque pas l'occlusion du tube. Dans ce cas, la solution reflue en amont de la pompe en raison de la surpression présente en aval, ce qui provoque une baisse de pression. La pression seuil ne peut alors pas être atteinte avant la fin du 1,1 tour programmé.

Si cette première partie du procédé est passée avec succès, l'unité de contrôle règle le débit de la pompe (3) sur une valeur intermédiaire, par exemple 100 ml/h. Le moteur est ensuite mis en marche pour 1,1 tour, opération qui dure environ 4 s. La pression instantanée est mesurée durant toute cette opération et les valeurs minimum et maximum sont calculées. A la fin de cette opération, les valeurs minimum $P_{min}$ et maximum $P_{max}$ de la pression instantanées doivent vérifier les équations (B) et (C) suivantes :

$$(B) \qquad (P_{min} - P_0) \geq P_2 \qquad \text{avec } P_2 = Q_2 \times (P_{1bar} - P_{0bar})$$

$$(C) \qquad (P_{max} - P_0) \geq P_3 \qquad \text{avec } P_3 = Q_3 \times (P_{1bar} - P_{0bar})$$

Les paramètres $Q_2$ et $Q_3$ sont déterminés statistiquement à l'aide de plusieurs pompes du même type. Ils sont respectivement de l'ordre de 0,6 et de 1,2. Si l'une de ces deux équations n'est pas vérifiée, une alarme est déclenchée. En effet, si la valeur minimum tombe en dessous du seuil $P_2$, cela indique une perte de charge. Si le seuil $P_3$ n'est pas atteint, il est probable que la pompe (3) a présenté lors de cette opération un défaut d'occlusion passé inaperçu lors de la première partie du contrôle en raison de la vitesse plus importante de rotation du moteur et de la pression plus faible régnant dans le tube.

Parallèlement, la pression instantanée ne doit à aucun moment dépasser la valeur seuil $P_4$ indicatrice d'une surpression excessive dans le système. Si l'équation (D) suivante est satisfaite, le moteur est arrêté et une alarme est générée :

$$(D) \qquad (P_i - P_0) \geq P_4 \qquad \text{avec } P_4 = Q_4 \times (P_{1bar} - P_{0bar})$$

Le paramètre $Q_4$ est déterminé statistiquement à l'aide de plusieurs pompes du même type. Il est de l'ordre de 2,5.

Ces différents critères sont regroupés dans le diagramme 2 présentant un exemple de courbe de pression pour un système ne présentant pas de défaut et un exemple de courbe de pression pour un système ayant un défaut d'occlusion.

Si l'ensemble du procédé est réalisé avec succès, l'unité de contrôle fait tourner la pompe en sens inverse du nombre exact de cycles qu'elle a réalisé lors des étapes précédentes, afin de supprimer la surpression régnant dans la section du tube (2) située entre la pompe (3) et le dispositif d'obturation (8) puis le dispositif d'obturation (8) est ouvert.

Lors de l'exécution du procédé de contrôle ainsi que durant la perfusion, le détecteur de pression (10) situé en amont de la pompe contrôle la pression régnant dans la section du tube située entre le récipient contenant la solution à administrer et la pompe. Il permet de détecter une occlusion ou une rupture de cette section du tube qui se traduisent toutes les deux par une diminution de la pression.

Le tube étant muni d'une pince automatique (11) en forme de flèche ne pouvant être introduite dans le boîtier de la pompe (3) que dans un sens, le risque d'inversement du positionnement du tube (2) dans la pompe (3) est considérablement diminué.

Si par ailleurs on connaît la longueur de la section du tube située entre le dernier doigt (7) de la pompe (3) et le dispositif d'obturation (8) ainsi que son diamètre et son élasticité, la mesure de $P_{max}$ permet de vérifier que le tube (2) positionné dans la pompe (3) correspond au tube programmé dans la pompe.

Si les équations (A), (B) ou (C) ne sont pas satisfaites, ceci indique

- soit une situation de sous-débit (under-flow) due par exemple à une occlusion du tube en amont de la pompe ou à un mauvais positionnement du tube (2) dans la pompe (3) ;
- soit une situation de surdébit (over-flow) due par exemple à une occlusion défectueuse dans une zone de la pompe (3) ou à un mauvais positionnement du tube (2) dans cette pompe (2).

La mesure permanente de la pression en amont et en aval de la pompe (3) à l'aide des détecteurs de pression (9) et (10) permet de détecter même au cours de la perfusion une occlusion du tube aussi bien entre le récipient contenant la solution et la pompe qu'entre la pompe et le patient. La rupture du tube en amont de la pompe est également détectée par le détecteur de pression (10).

La présence d'un dispositif tel que celui de la pince automatique (11) empêche une inversion du sens du tube (2) dans la pompe (3).

Le procédé conforme à l'invention permet donc de remplir les mêmes fonctions que le détecteur de gouttes de l'état de la technique sans en avoir les inconvénients.

Liste des références :

(1) Dispositif de mise en oeuvre du procédé
(2) Tube souple
(3) Pompe à doigts
(4) Moteur de la pompe
(5) Plaque d'appui de la pompe
(6) Arbre à cames
(7) Doigts de la pompe
(8) Dispositif d'obturation du tube
(9) Détecteur de pression en aval de la pompe
(10) Détecteur de pression en amont de la pompe
(11) Pince automatique en forme de flèche

(21) Moteur du dispositif d'obturation du tube
(22) Tête du dispositif d'obturation du tube
(23) Dispositif à vis
(24) Boîtier du dispositif d'obturation du tube
(25) Anneau en caoutchouc

**Revendications**

1. Procédé de contrôle d'un dispositif de pompage comportant une pompe à doigts munie d'un tube souple **caractérisé en ce que** le tube souple (2) est obturé à l'aide d'un dispositif d'obturation (8) situé en aval de la pompe (3), puis la pompe (3) est mise en marche et la pression instantanée $P_I$ est mesurée dans la section du tube (2) située entre la pompe (3) et le dispositif d'obturation (8).

2. Procédé selon la revendication 1, **caractérisé en ce que** les étapes suivantes sont réalisées:

   a) la pompe (3), réglée sur un débit proche du débit maximum, effectue un nombre de cycles prédéterminé au moins égal à 1 puis elle est arrêtée ;
   b) une alarme est déclenchée si la pression instantanée $P_I$, mesurée tout au long de l'étape a), n'atteint pas une valeur seuil $P_1$ au plus tard à la fin de l'étape précédente.

3. Procédé selon la revendication 2, **caractérisé en ce que** les étapes suivantes sont réalisées si aucune alarme n'est déclenchée lors de l'étape b):

   c) la pompe (3), réglée sur un débit intermédiaire, effectue un nombre de cycles prédéterminé au moins égal à 1 puis elle est arrêtée ;
   d) la pression minimum $P_{min}$ et la pression maximum $P_{max}$ atteintes lors de l'étape précédente sont mesurées ;
   e) une alarme est déclenchée si la pression minimum $P_{min}$ ou la pression maximum $P_{max}$ déterminées à l'étape d) n'atteignent pas les valeurs seuil $P_2$ et $P_3$ respectivement.

4. Procédé selon la revendication 3, **caractérisé en ce que**, dans une étape f), une alarme est déclenchée si la pression instantanée $P_I$ atteint une valeur seuil $P_4$ au cours de l'étape c).

5. Procédé selon l'une des revendications précédentes, **caractérisé en ce que**, dans une étape g), la pompe (3) effectue en sens inverse le nombre exact de cycles qu'elle a réalisé lors des étapes précédentes puis le dispositif d'obturation (8) est rouvert si à la fin du procédé aucune alarme n'a été déclenchée.

**6.** Dispositif de contrôle pour la mise en oeuvre du procédé selon l'une des revendications précédentes, **caractérisé en ce qu'**il comporte un tube souple (2), une pompe à doigts (3) dans laquelle le tube souple (2) est introduit, un dispositif d'obturation (8) situé en aval de la pompe (3) et un détecteur de pression (9) situé entre la pompe (3) et le dispositif d'obturation (8).

**7.** Dispositif de contrôle selon la revendication 6, **caractérisé en ce qu'il** comporte un détecteur de pression (10) situé en amont de la pompe.

**8.** Dispositif de contrôle selon l'une des revendications 6 ou 7, **caractérisé en ce qu'**il comporte une unité de commande permettant le contrôle de l'ouverture et de la fermeture du dispositif d'obturation (8), le réglage du débit de la pompe (3) et la mise en marche de la pompe (3) pendant le nombre de cycles déterminé lors de l'étape a), l'analyse des valeurs mesurées de la pression instantanée $P_i$ pour l'étape b), l'arrêt de la pompe (3) lorsqu'une alarme est générée.

**9.** Dispositif de contrôle selon la revendication 8, **caractérisé en ce que** l'unité de commande permet le réglage du débit de la pompe (3) et la mise en marche de la pompe (3) pendant le nombre de cycles déterminé lors de l'étape c), l'analyse des valeurs mesurées de la pression instantanée $P_I$ pour les étapes d), e) et f), l'arrêt de la pompe (3) lorsqu'une alarme est générée et permet de faire effectuer en sens inverse à la pompe (3) autant de cycles qu'il y en a eu lors des étapes précédentes.

**10.** Dispositif de contrôle selon l'une des revendications 6 à 9, **caractérisé en ce que** le tube (2) ne peut être introduit dans la pompe (3) que dans un sens prédéterminé.

**Claims**

**1.** Method of controlling a pump comprising a finger pump provided with a flexible tube, **characterised in that** the flexible tube (2) is closed off by means of a closure device (8) situated downstream of the pump (3), and then the pump (3) is started and the instantaneous pressure $P_I$ is measured in the section of the tube (2) situated between the pump (3) and the closure device (8).

**2.** Method according to claim 1, **characterised in that** the following steps are performed:

a) the pump (3), adjusted to an output close to the maximum output, performs a predetermined number of cycles at least equal to 1, and is then stopped;
b) an alarm is triggered if the instantaneous pressure $P_I$, measured throughout step a), does not reach a threshold value $P_1$ at the latest by the end of the previous step.

**3.** Method according to claim 2, **characterised in that** the following steps are performed if no alarm is triggered during step b):

c) the pump (3), adjusted to an intermediate output, performs a predetermined number of cycles at least equal to 1 and is then stopped;
d) the minimum pressure $P_{min}$ and the maximum pressure $P_{max}$ reached during the previous step are measured;
e) an alarm is triggered if the minimum pressure $P_{min}$ or the maximum pressure $P_{max}$ determined at step d) do not reach the threshold values $P_2$ and $P_3$ respectively.

**4.** Method according to claim 3, **characterised in that**, in a step f), an alarm is triggered if the instantaneous pressure $P_I$ reaches a threshold value $P_4$ during step c).

**5.** Method according to one of the preceding claims, **characterised in that**, in a step g), the pump (3) performs, in the opposite direction, the exact number of cycles that it performed during the previous steps and then the closure device (8) is opened if at the end of the method no alarm has been triggered.

**6.** Control device for implementing the method according to one of the preceding claims, **characterised in that** it comprises a flexible tube (2), a finger pump (3) into which the flexible tube (2) is introduced, a closure device (8) situated downstream of the pump (3) and a pressure detector (9) situated between the pump (3) and the closure device (8).

7. Control device according to claim 6, **characterised in that** it comprises a pressure detector (10) situated upstream of the pump.

8. Control device according to one of claims 6 or 7, **characterised in that** it comprises a control unit for controlling the opening and closing of the closure device (8), adjusting the output of the pump (3) and starting the pump (3) during the number of cycles determined during step a), analysing the measured values of the instantaneous pressure $P_I$ for step b), and stopping the pump (3) when an alarm is generated.

9. Control device according to claim 8, **characterised in that** the control unit enables the output of the pump (3) to be adjusted and the pump (3) to be started during the number of cycles determined during step c), the measured values of the instantaneous pressure $P_I$ for steps d), e) and f) to be analysed, and the pump (3) to be stopped when an alarm is generated, and makes it possible to make the pump (3) perform, in the opposite direction, as many cycles as there have been during the previous steps.

10. Control device according to one of claims 6 to 9, **characterised in that** the tube (2) can be introduced into the pump (3) only in a predetermined direction.

**Patentansprüche**

1. Verfahren zur Steuerung einer Pumpvorrichtung, welche eine Fingerpumpe aufweist, die mit einem elastischen Schlauch ausgestattet ist, **dadurch gekennzeichnet, dass** der elastische Schlauch (2) mittels einer Abdichtvorrichtung (8) abgedichtet wird, die abwärts der Pumpe (3) angeordnet ist, dann die Pumpe (3) in Gang gesetzt wird und der momentane Druck $P_i$ in dem Abschnitt des Schlauchs (2), der sich zwischen der Pumpe (3) und der Abdichtvorrichtung (8) befindet, gemessen wird.

2. Verfahren gemäß Anspruch 1, **dadurch gekennzeichnet, dass** die folgenden Schritte ausgeführt werden:

   a) die Pumpe (3), die auf einen Durchsatz nahe dem maximalen Durchsatz eingestellt ist, führt eine vorbestimmte Anzahl Zyklen von wenigstens gleich 1 aus und wird dann angehalten;
   b) es wird ein Alarm ausgelöst, wenn der momentane Druck $P_i$, der während des gesamten Schritts a) gemessen wird, nicht spätestens am Ende des vorausgegangenen Schritts einen Schwellenwert $P_1$ erreicht.

3. Verfahren gemäß Anspruch 2, **dadurch gekennzeichnet, dass** die folgenden Schritte ausgeführt werden, wenn bei Schritt b) kein Alarm ausgelöst wird:

   c) die Pumpe (3), die auf einen mittleren Durchsatz eingestellt ist, führt eine vorbestimmte Anzahl Zyklen von wenigstens gleich 1 aus und wird dann angehalten;
   d) der minimale Druck $P_{min}$ und der maximale Druck $P_{max}$, die bei dem vorausgegangenen Schritt erreicht wurden, werden gemessen;
   e) es wird ein Alarm ausgelöst, wenn der minimale Druck $P_{min}$ und der maximale Druck $P_{max}$, die bei Schritt d) bestimmt wurden, nicht die jeweiligen Schwellenwerte $P_2$ und $P_3$ erreichen.

4. Verfahren gemäß Anspruch 3, **dadurch gekennzeichnet, dass** in einem Schritt f) ein Alarm ausgelöst wird, wenn der momentane Druck $P_i$ im Laufe von Schritt c) einen Schwellenwert $P_4$ erreicht.

5. Verfahren gemäß einem der vorausgegangenen Ansprüche, **dadurch gekennzeichnet, dass** in einem Schritt g) die Pumpe (3) die gleiche Anzahl Zyklen, die sie bei den vorausgegangenen Schritten ausgeführt hat, in umgekehrter Richtung ausführt, dann die Abdichtvorrichtung (8) wieder geöffnet wird, wenn am Ende des Verfahrens kein Alarm ausgelöst wurde.

6. Vorrichtung für die Durchführung des Verfahrens gemäß einem der vorausgegangenen Ansprüche, **dadurch gekennzeichnet, dass** sie einen elastischen Schlauch (2), eine Fingerpumpe (3), in welche der elastische Schlauch (2) eingesetzt wird, eine abwärts der Pumpe (3) angeordnete Abdichtvorrichtung (8) und einen Druckdetektor (9) aufweist, der zwischen der Pumpe (3) und der Abdichtvorrichtung (8) angeordnet ist.

7. Steuervorrichtung gemäß Anspruch 6, **dadurch gekennzeichnet, dass** sie einen Druckdetektor (10) aufweist, der aufwärts der Pumpe angeordnet ist.

8. Steuervorrichtung gemäß einem der Ansprüche 6 oder 7, **dadurch gekennzeichnet, dass** sie eine Steuereinheit aufweist, welche die Steuerung des Öffnens und des Schließens der Abdichtvorrichtung (8), das Einstellen des Durchsatzes der Pumpe (3) und das in Gang Setzen der Pumpe (3) während der bei Schritt a) bestimmten Anzahl von Zyklen, die Analyse der gemessenen Werte des momentanen Drucks $P_i$ für Schritt b), das Anhalten der Pumpe (3), wenn ein Alarm erzeugt wird, ermöglicht.

9. Steuervorrichtung gemäß Anspruch 8, **dadurch gekennzeichnet, dass** die Steuereinheit das Einstellen des Durchsatzes der Pumpe (3) und das in Gang Setzen der Pumpe (3) während der bei Schritt c) bestimmten Anzahl von Zyklen, die Analyse der gemessenen Werte des momentanen Drucks $P_i$ für die Schritte d), e) und f), das Anhalten der Pumpe (3), wenn ein Alarm erzeugt wird, ermöglicht, sowie ermöglicht, dass die Pumpe (3) genauso viele Zyklen in umgekehrter Richtung ausführt, wie sie in den vorausgegangenen Schritten ausgeführt hat.

10. Steuervorrichtung gemäß einem der Ansprüche 6 bis 9, **dadurch gekennzeichnet, dass** der Schlauch (2) nur in einer vorbestimmten Richtung in die Pumpe (3) eingesetzt werden kann.

## Figure 1

## Figure 2

# Diagramme 1

ON/OFF

Tension

ts

ton

# Diagramme 2

bars

pas de problème
d'occlusivité

problème d'occlusivité (bloc mécanique
de pompage mal vissé)

secondes

## RÉFÉRENCES CITÉES DANS LA DESCRIPTION

**Documents brevets cités dans la description**

- EP 0315312 A
- US 4840542 A
- FR 9901132